# EUROPEAN PATENT SPECIFICATION

(11) **EP 3 432 959 B1**
(45) Date of publication and mention of the grant of the patent: **06.05.2020**
(21) Application number: 17714518.2
(22) Date of filing: 21.03.2017
(51) Int. Cl.: A61M 15/06, A24F 47/00, A61M 11/04, A61M 15/00

(54) **VAPOUR PROVISION DEVICE**
DAMPFBEREITSTELLUNGSVORRICHTUNG
DISPOSITIF DE FOURNITURE DE VAPEUR

(30) Priority: 24.03.2016 GB 201605103; 21.07.2016 GB 201612685
(43) Date of publication of application: 30.01.2019
(73) Proprietor: Nicoventures Holdings Limited, London WC2R 3LA (GB)
(72) Inventor: NETTENSTROM, Matthew Joel, London WC2R 3LA (GB); LEADLEY, David, London WC2R 3LA (GB); SCHENNUM, Steven Michael, London WC2R 3LA (GB); JAIN, Siddhartha, London WC2R 3LA (GB)
(74) Representative: D Young & Co LLP
(86) International application number: PCT/GB2017/050787
(87) International publication number: WO 2017/163050

(56) References cited:
- EP-A2- 2 801 270
- WO-A1-2016/014652
- US-A1- 2015 027 457

## Description

### Field

The present disclosure relates to a vapour provision device, e.g. an e-cigarette.

### Background

Many electronic vapour provision systems, such as e-cigarettes and other electronic nicotine delivery systems, are formed from two main components - a cartomiser and a control unit. The cartomiser generally includes a reservoir of liquid and an atomiser for vaporising the liquid. The atomiser is often implemented as an electrical (resistive) heater, such as a coil of wire. The control unit generally includes a battery for supplying power to the atomiser. In operation, the control unit may be activated, for example by detecting when a user inhales on the device and/or when the user presses a button, to provide electrical power from the battery to the heater. This activation causes the heater to vaporise a small amount of liquid from the reservoir, which is then inhaled by the user.

This type of e-cigarette therefore generally incorporates two consumables, firstly the liquid to be vaporised, and secondly power in the battery. Regarding the former, once the reservoir of liquid has been exhausted, the cartomiser may be discarded to allow replacement with a new cartomiser. Regarding the latter, the control unit may provide some form of electrical connector for receiving power from an external source, thereby allowing the battery within the e-cigarette to be re-charged.

Although e-cigarettes have developed rapidly over the past few years, there remain areas where it is desirable to improve the operability and user experience for such devices.

US 2015/027457 discloses an apparatus and method for controlling resistance-to-draw of an electronic smoking article. The apparatus includes a reusable portion and a cartomizer portion which are configured to supply an air flow from one or more inlets in an outer cylindrical housing of the electronic smoking article to a cartomizer via a cartomizer inlet having a fixed diameter configured to control a resistance-to-draw of the electronic smoking article. The cartomizer inlet is located inside the outer cylindrical housing of the electronic smoking article, wherein a combined air flow area of the one or more inlets in the outer housing of the electronic smoking article is greater than a cross-sectional area of the cartomizer inlet. The method includes heating a liquid material from a reservoir to form an aerosol, and combining the at least initially volatilized liquid material with the air flow from the cartomizer inlet.

### Summary

The invention is defined in the appended claims.

Some embodiments provide a vapour provision device including an atomiser for vaporising a liquid; an air passage through the atomiser, the air passage exiting the device at a mouthpiece; at least one air inlet, joined by a channel to the air passage through the atomiser; and at least one resilient seal which acts to restrict / prevent air from the air inlet travelling to the air passage except through the channel. The seal comprises a resilient material and the size of the channel is defined at least in part by the resilient material to control resistance to draw for the vapour provision device.

### Brief Description of the Drawings

Various embodiments of the invention will now be described in detail by way of example only with reference to the following drawings:
Figure 1 is a cross-section through an e-cigarette comprising a cartomiser and a control unit in accordance with some embodiments of the invention.
Figure 2 is an isometric external view of the cartomiser of the e-cigarette of Figure 1 in accordance with some embodiments of the invention.
Figure 3 is a collection of five external views of the cartomiser of Figure 2 in accordance with some embodiments of the invention. In particular, the bottom view shows the cartomiser from underneath, the top view shows the cartomiser from above, the central view shows a face view of the cartomiser (from front or back), and on either side of the central view are respective side views of the cartomiser.
Figure 4 is an exploded view of the cartomiser of the e-cigarette of Figure 1 in accordance with some embodiments of the invention.
Figures 5A, 5B and 5C illustrate the wick/heater assembly being fitted into the cartomiser plug in accordance with some embodiments of the invention.
Figures 6A and 6B illustrate the inner frame and the vent seal being fitted into the cartomiser plug in accordance with some embodiments of the invention.
Figures 7A and 7B illustrate the combination of the inner frame, wick/heater assembly, and primary seal being fitted into the shell and the reservoir then being filled with e-liquid in accordance with some embodiments of the invention.
Figures 8A and 8B illustrate the PCB and end cap being fitted to the other components to complete the formation of the cartomiser in accordance with some embodiments of the invention.
Figure 9 is a top view looking down onto the control unit of the e-cigarette of Figure 1 in accordance with some embodiments of the invention.
Figures 10A and 10B are cross-sections respectively (a) from side to side, and (b) from front to back, showing the airflow through the e-cigarette of Figure 1 in accordance with some embodiments of the invention.
Figure 11A shows two alternative implementations of the battery seal in accordance with some embodiments of the invention, and Figure 11B shows a cross-section through a channel formed in each battery seal of Figure 11A.

### Detailed Description

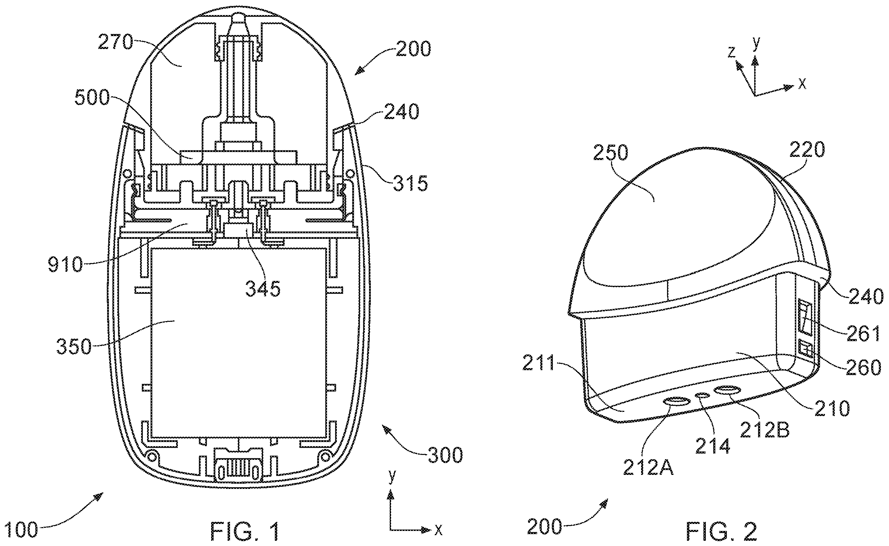

Figure 1 is a cross-section through an e-cigarette 100 in accordance with some embodiments of the invention. The e-cigarette comprises two main components, namely a cartomiser 200 and a control unit 300. As discussed in more detail below, cartomiser includes a chamber 270 containing a reservoir of liquid, a heater to act as an atomiser or vaporiser, and a mouthpiece. The liquid in the reservoir (sometimes referred to as the e-liquid) typically includes nicotine in an appropriate solvent, and may include further constituents, for example, to aid aerosol formation, and/or for additional flavouring. The cartomiser 200 further includes a wick/heater assembly 500, which includes a wick or similar facility to transport a small amount of liquid from the reservoir to a heating location on or adjacent the heater. The control unit 300 includes a re-chargeable cell or battery 350 to provide power to the e-cigarette 100, a printed circuit board (PCB) for generally controlling the e-cigarette (not shown in Figure 1), and a microphone 345 for detecting a user inhalation (via a pressure drop). When the heater receives power from the battery, as controlled by the PCB in response to the microphone 345 detecting a user puff on the e-cigarette 100, the heater vaporises the liquid from the wick and this vapour is then inhaled by a user through the mouthpiece.

For ease of reference, the x and y axes are marked in Figure 1. The x axis will be referred to herein as the width of the device (from side to side), while the y axis will be referred to herein as the height axis, where the cartomiser 200 represents the upper portion of the e-cigarette 100 and the control unit 300 represents the lower portion of the e-cigarette. Note that this orientation reflects how a user holds the e-cigarette 100 during normal operation of the device, given that the wick is located in the lower part of the reservoir in the cartomiser 200. Therefore holding the e-cigarette 100 in this orientation ensures that the wick is in contact with liquid at the bottom of the reservoir.

We further assume a z axis (not shown in Figure 1) which is perpendicular to the x and y axes shown in Figure 1. The z axis will be referred to herein as the depth axis. The depth of e-cigarette 100 is significantly less than the width of the e-cigarette, thereby resulting in a generally flat or planar configuration (in the x-y plane). Accordingly, the z axis can be considered as extending from face to face of the e-cigarette 100, where one face may be regarded (arbitrarily) as the front face of the e-cigarette and the opposing face as the back face of the e-cigarette 100.

The cartomiser 200 and the control unit 300 are detachable from one another by separating in a direction parallel to the y-axis, but are joined together when the device 100 is in use so as to provide mechanical and electrical connectivity between the cartomiser 200 and the control unit 300. When the e-liquid in cartomiser reservoir 270 has been depleted, the cartomiser 200 is removed and a new cartomiser is attached to the control unit 300. Accordingly, the cartomiser 200 may sometimes be referred to as the disposable portion of the e-cigarette 100, while the control unit 300 represents the re-usable portion.

Figure 2 is an isometric external view of the cartomiser of the e-cigarette of Figure 1 in accordance with some embodiments of the invention. This external view confirms that the depth of the cartomiser 200 (and the e-cigarette 100 as a whole), as measured parallel to the z axis, is significantly less than the width of the cartomiser 200 (and the e-cigarette 100 as a whole), as measured parallel to the x axis. Note that overall, the external appearance of the cartomiser 200 is relatively smooth and uncluttered.

The cartomiser 200 comprises two main portions (at least from an external viewpoint). In particular, there is a lower or base portion 210 and an upper portion 220. The upper portion 220 provides the mouthpiece 250 of the e-cigarette, as described in more detail below. When the cartomiser 200 is assembled with the control unit 300, the base portion 210 of the cartomiser sits within the control unit 300, and hence is not externally visible, whereas the upper portion 220 of the cartomiser protrudes above the control unit 300, and hence is externally visible. Accordingly, the depth and width of the base portion 210 are smaller than the depth and width of the upper portion 220, to allow the base portion to fit within the control unit 300. The increase in depth and width of the upper portion 220 compared with the base portion 210 is provided by a lip or rim 240. When the cartomiser 200 is inserted into the control unit 300, this lip or rim 240 abuts against the top of the control unit.

As shown in Figure 2, the side wall of base portion 210 includes a notch or indentation 260 for receiving a corresponding latching member from the control unit 300. The opposite side wall of the base portion 210 is provided with a similar notch or indentation to likewise receive a corresponding latching member from the control unit 300. It will be appreciated that this pair of notches 260 on the base portion 200 (and the corresponding latching members of the control unit) provide a latch or snap fit connection for securely retaining the cartomiser 200 within the control unit 300 during operation of the device. Adjacent to the notch 260 is a further notch or indentation 261, which is utilised in the formation of the cartomiser 200, as described in more detail below.

As also shown in Figure 2, the bottom wall 211 of the base portion 210 includes two larger holes 212A, 212B on either side of a smaller hole 214 for air inlet. The larger holes 212A and 212B are used to provide positive and negative electrical connections from the control unit 300 to the cartomiser 200. Thus when a user inhales through the mouthpiece 250 and the device 100 is activated, air flows into the cartomiser 200 through the air inlet hole 214. This incoming air flows past the heater (not visible in Figure 2), which receives electrical power from the battery in the control unit 300 so as to vaporise liquid from the reservoir (and more especially from the wick). This vaporised liquid is then incorporated or entrained into the airflow through the cartomiser, and hence is drawn out of the cartomiser 200 through mouthpiece 250 for inhalation by the user.

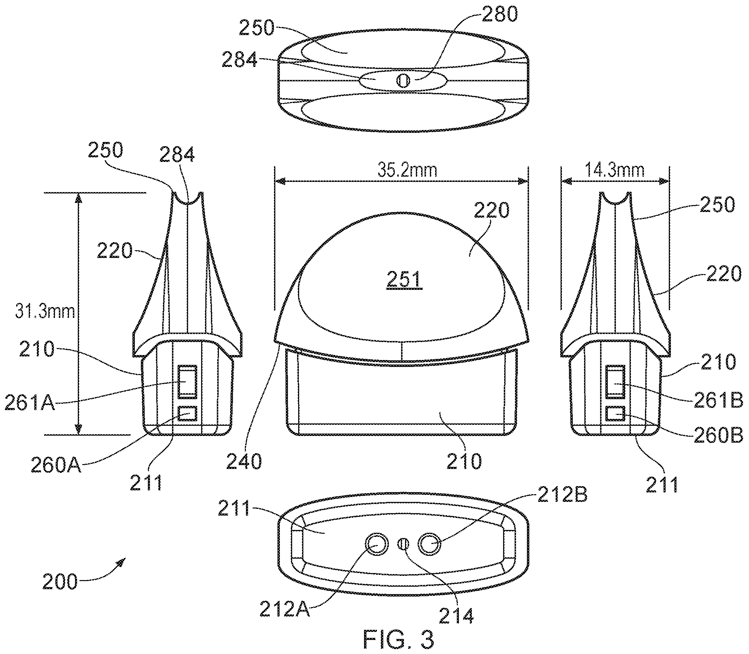

Figure 3 is a collection of five external views of the cartomiser 200 of Figure 2 in accordance with some embodiments of the invention. In particular, the bottom view shows the cartomiser from underneath, the top view shows the cartomiser from above, the central view shows a face view of the cartomiser (from front or back), and on either side of the central view are respective side views of the cartomiser. Note that since the cartomiser is symmetric front/back (i.e. with respect to the z axis), the front face of the cartomiser and the back face of the cartomiser both correspond to the central view of Figure 3. In addition, the cartomiser is also symmetric in the width direction (i.e. with respect to the x axis), hence the two side views to the left and right of the central view are the same.

Figure 3 illustrates the various features of the cartomiser already discussed above with respect to Figure 2. For example, the central view clearly shows the top portion 220 and the bottom portion 210 of the cartomiser. The lower view shows the bottom wall of the base portion 211, including the two larger holes 212A and 212B, which are used to provide positive and negative electrical connections from the control unit 300 to the cartomiser 200, plus the smaller hole 214 for air inlet into the cartomiser. In addition, the two side views show the two notches in each side wall, an upper notch 261A, 261B, and a lower notch 260A, 260B, the latter being used to fasten the cartomiser 200 to the control unit 300.

The top view further shows a hole 280 in the mouthpiece 250 which represents the air outlet from the cartomiser 200. Thus in operation, when a user inhales, air enters the cartomiser at the bottom through inlet 214, flows through the atomiser, including past the heater, where it acquires vapour, and then travels up the centre of the cartomiser to exit through air outlet 280.

Figure 3 provides dimensions of the cartomiser 200, showing a maximum height (in the y direction) of 31.3mm, a maximum width (in the x direction) of 35.2mm, and a maximum depth of 14.3 mm (parallel to the z direction). Note that these maximum width and depth measurements relate to the upper portion 220 of the cartomiser; the width and depth of the base portion 210 are somewhat smaller, in order to allow the base portion to be received into the control unit 300. The difference in width and depth between the upper portion 220 and the base portion 210 is accommodated by the rim or flange 240, as described above.

It will be appreciated that the dimensions shown in Figure 3 are provided by way of example only, and may vary between embodiments. Nevertheless, the dimensions given do confirm that the e-cigarette 100, including the cartomiser, has an approximately flat or planar shape, with one relatively small dimension (the z direction) perpendicular to the planar shape. This planar shape is extended by the control unit 300, which in effect extends the height (y dimension of the cartomiser), but shares substantially the same width and depth.

Figure 3 also gives a clear indication of the size and shape of the mouthpiece 250. In contrast to many e-cigarettes, which provide a circular mouthpiece akin to a straw or conventional cigarette, the mouthpiece 250 has a very different and distinctive shape. In particular, the mouthpiece comprises a pair of large, relatively flat, opposing faces. One of these mouthpiece faces is denoted as face 251 in the central view of Figure 3, and there is a corresponding, opposing face to the rear of the device. (Note that the labelling of front and back for the cartomiser is arbitrary, since it is symmetric with respect to the z axis, and can be fitted either way around onto the control unit 300).

The front and rear faces provide relatively large surfaces onto which the lips of a user can be placed. For example, we can consider the front face to provide a surface for engaging the upper lip, and the rear face to provide a surface for engaging the lower lip. In this configuration, we can regard the height (y axis) of the e-cigarette 100 defining a longitudinal axis extending away from the user's mouth, the width of the e-cigarette 100 (the x axis) as running parallel to the line between a user's upper and lower lips, and the depth of the e-cigarette 100 (the z axis) as running parallel to the direction of separation of the user's upper and lower lips.

The height of the front and rear mouthpiece faces (approximately 17 mm in the particular embodiment of Figure 3) is broadly comparable to the typical thickness of a lip, and therefore large enough to readily accommodate in this direction a lip placed on the surface. Similarly, the width of the front and rear mouthpiece faces (approximately 28 mm in the particular embodiment of Figure 3) represents a significant proportion (very approximately half) of the typical width of lips (from one side of the mouth to the other).

This shape and sizing of the mouthpiece 250 allows the lips of user to engage the mouthpiece for inhalation with much less distortion from the normal resting position of the mouth - e.g. there is no need to purse the lips, as for a straw or conventional cigarette having a small circular mouthpiece. This makes using the mouthpiece 250 of the e-cigarette 100 a more relaxing experience, and also may help to ensure a more consistent seal between the mouth and the mouthpiece.

In addition, e-cigarette 100 (like many other e-cigarettes) uses a sensor to detect airflow through the device, i.e. a user puff, which can then trigger operation of the heater to vaporise the liquid. The device has to discriminate between the airflow caused by a user puff, and other forms of airflow or pressure changes that arise due to other actions or circumstances - e.g. movement of the e-cigarette through the air, being on a railway train which enters a tunnel etc. Having a consistent seal between the mouth and the mouthpiece 250 can help the device provide better discrimination of an actual inhalation, and so reduce the risk of unintentional activation of the heater.

Furthermore, some e-cigarettes use sensor measurements of the airflow through the device not only to initiate activation of the heater, but also to provide dynamic control of the heater (or other components of the e-cigarette). For example, as the measured airflow increases, the heater may be provided with more power, firstly to compensate for the cooling effect of the increased airflow, and/or secondly to vaporise more liquid into the increased airflow. Having a consistent seal between the mouth and the mouthpiece 250 can again help to improve the reliability and accuracy of this dynamic control.

In addition, with reference to the side views of Figure 3, it can be seen that the front and back faces of the mouthpiece generally slope towards one another at the top of the device. In other words, the depth or separation of the opposing faces (as measured in the z direction) decreases towards the air outlet hole 280 (i.e. as the y axis increases). This slope is relatively gentle - approximately 15 degrees with respect to the y axis. This incline helps to provide a natural and comfortable engagement between the faces of the mouthpiece 251 and the lips of a user.

As can be seen in Figure 3, the front and back faces 251 do not converge completely at the top of the mouthpiece, but rather overhang to provide a small valley 284 which extends in the x-direction of the device. The opening 280, which allows air and vapour to exit from the cartomiser 200, is formed in the centre of this valley 284. Having this small overhang, so that the mouthpiece opening 280 is located in the groove or valley 284, helps to protect the mouthpiece opening from physical contact, and hence from potential damage and dirt.

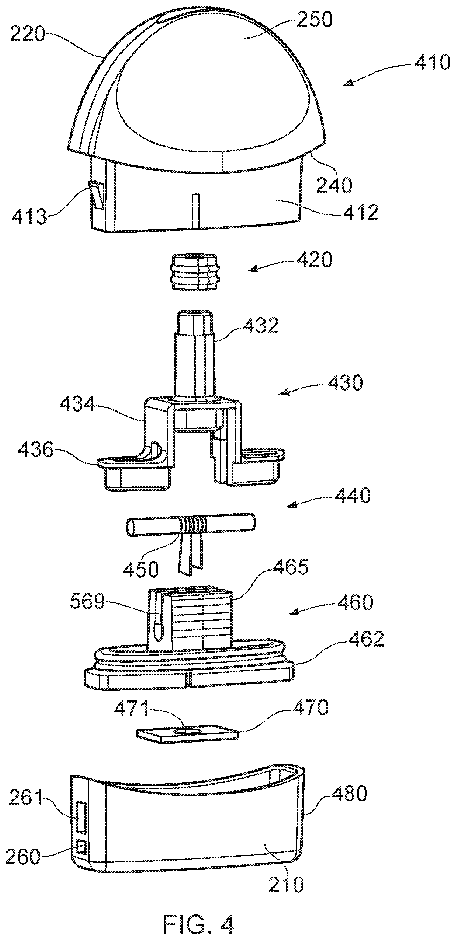

Figure 4 is an exploded view of the cartomiser 200 of the e-cigarette of Figure 1 in accordance with some embodiments of the invention. The cartomiser includes a shell 410, a vent seal 420, an inner frame 430, a heating coil 450 located on a wick 440, a primary seal 460 (also referred to as the cartomiser plug), a printed circuit board (PCB) 470 and an end cap 480. The view of Figure 4 shows the above components exploded along the longitudinal (height or y) axis of the cartomiser 200.

The cap 480 is formed from substantially rigid plastic such as polypropylene and provides the base portion 210 of the cartomiser. The cap is provided with two holes 260, 261 on each side (only one side is visible in Figure 4, but the side which is not visible is the same as the side that is visible). The lower hole 260 is for latching the cartomiser 200 to the control unit 300, while the upper hole 261 is for latching the end cap 480 to the shell 410. As described in more detail below, latching the cap 480 and the shell 410 in effect completes the assembly of the cartomiser, and retains the various components shown in Figure 4 in the correct position.

Above the end cap is located the PCB 470, which includes a central air hole 471 to allow air to flow through the PCB into the atomiser (the end cap 480 is likewise provided with a central air hole, not visible in Figure 4) to support this air flow into the atomiser. In accordance with some embodiments, the PCB does not contain any active electrical components, but rather provides a circuit or conductive path between the control unit 300 and the heater 450.

Above the PCB 470 is located the primary seal 460, which has two main portions, an upper portion which defines (in part) an atomizer chamber 465, and a lower portion 462 which acts as an end seal for the reservoir 270. Note that in the assembled cartomiser 200, the reservoir of e-liquid is located around the outside of the atomizer chamber, and the e-liquid is prevented from leaving the cartomiser (at least in part) by the lower portion 462 of the cartomiser plug 460. The cartomiser plug is made from a material that is slightly deformable. This allows the lower portion 462 to be compressed a little when inserted into the shell 410, and hence provide a good seal to retain the e-liquid in reservoir 270.

Two opposing side walls of the atomiser chamber 465 are provided with respective slots 569 into which the wick 440 is inserted. This configuration thereby ensures that the heater 450, which is positioned on the wick, is located near the bottom of the atomiser chamber to vaporise liquid introduced into the atomiser chamber 465 by wick 440. In some embodiments, the wick 440 is made of glass fibre rope (i.e. filaments or strands of glass fibre twisted together), and the heater coil 450 is made of nichrome (an alloy of nickel and chromium). However, various other types of wick and heater are known and could be used in the cartomiser 200, such as a wick made out of porous ceramic, and/or some form of planar heater (rather than a coil). Note that although Figure 4 suggests that the heater coil 450 has a loop of wire dropping down from the wick at each end, in practice there is just a single lead at each end (as described in more detail below).

The cartomiser plug 460 and the wick/heater assembly are surmounted by the inner frame 430, which has three main sections. The inner frame is substantially rigid, and may be made of a material such as polybutylene terephthalate. The lowermost section 436 of the inner frame 430 covers the lower portion 462 of the cartomiser plug 460, while the middle section 434 completes the atomiser chamber 465 of the cartomiser plug. In particular, the inner frame provides the top wall of the atomiser chamber, and also two side walls that overlap with the two side walls of the atomising chamber 465 of the cartomiser plug. The final section of the inner frame is an airflow tube 432 that leads upwards from the top wall of the atomising chamber (part of the middle section 434) and connects with the mouthpiece hole 280. In other words, tube 432 provides a passage for vapour produced in the atomising chamber 465 to be drawn out of the e-cigarette 100 and inhaled through mouthpiece 250.

Since the inner frame is substantially rigid, the vent seal 420 is provided at (inserted around) the top of the airflow tube 432 to ensure a proper seal between the inner frame and the mouthpiece exit hole 280. The vent seal 420 is made of a suitably deformable and resilient material such as silicone. Lastly, the shell 410 provides the external surface of the upper portion 220 of the cartomiser 200, including the mouthpiece 250, and also the lip or flange 240. The shell 410, like the end cap, is formed of a substantially rigid material, such as polypropylene. The lower section 412 of the shell 410 (i.e. below the lip 240) sits inside the end cap 480 when the cartomiser has been assembled. The shell is provided with a latch tab 413 on each side to engage with hole 261 on each side of the end cap 480, thereby retaining the cartomiser 200 in its assembled condition.

The airflow passage through the cartomiser enters a central hole in the cap 480 (not visible in Figure 4) and then passes through a hole 471 in the PCB. The airflow next passes up into the atomiser chamber 465, which is formed as part of the cartomiser plug 460, flows around the wick and heater assembly 500 and through the tube 432 of the inner frame 430 (and through vent seal 420), and finally exits through the hole 280 in the mouthpiece 250.

The reservoir 270 of e-liquid is contained in the space between this airflow passage and the outer surface of the cartomiser 200. Thus shell 410 provides the outer walls (and top) of the housing for the reservoir 270, while the lower section 436 of the inner frame in conjunction with the base portion 462 of the primary seal 460 and end cap 480 provide the bottom or floor of the housing for the reservoir of e-liquid. The inner walls of this housing are provided by the atomising chamber 465 of the primary seal 460, in cooperation with the middle section 434 of the inner frame, and also the airflow tube 432 of the inner frame 430 and the vent seal 420. In other words, the e-liquid is stored in the reservoir space between the outer walls and the inner walls. However, the e-liquid should not penetrate inside the inner walls, into the airflow passage, except via wick 440, otherwise there is a risk that liquid would leak out of the mouthpiece hole 280.

The capacity of this space is typically of the order of 2ml in accordance with some embodiments, although it will be appreciated that this capacity will vary according to the particular features of any given design. Note that unlike for some e-cigarettes, the e-liquid reservoir 270 is not provided with any absorbent material (such as cotton, sponge, foam, etc) for holding the e-liquid. Rather, the reservoir chamber only contains the liquid, so that the liquid can move freely around the reservoir 270. This has certain advantages, such as generally supporting a larger capacity, and also making the filling procedure less complex. One potential disadvantage with having a free liquid in the reservoir (i.e. not holding the liquid in a sponge or other absorbent structure) is that the liquid can flow more easily, and hence might be more likely to leak in an undesirable manner from the reservoir 270 into the airflow passage. However, such leakage is generally prevented by the vent seal 420 and the primary seal 460.

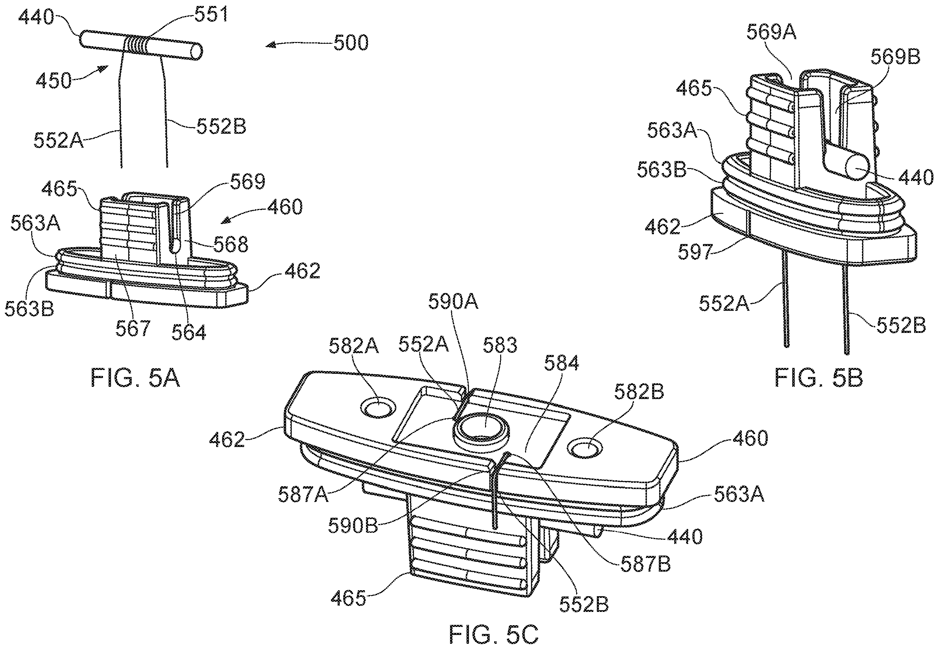

Figure 5A, 5B and 5C illustrate the wick/heater assembly being fitted into the cartomiser plug in accordance with some embodiments of the invention. The wick/heater assembly 500 is formed from the heater wire 450 and the wick 440. As noted above, the wick comprises glass fibres formed into a generally cylindrical or rod shape. The heater 450 comprises a coil of wire 551 wound around the wick. At each end of the coil there is a contact wire 552A, 552B, which together act as the positive and negative terminals to allow the coil to receive electrical power.

As visible in Figure 5A, the primary seal 460 includes the base portion 462 and the atomising chamber 465. The base portion is provided with two outwardly directed ribs. When the shell 410 is fitted over the base portion, these ribs are compressed slightly in order to fit inside the shell 410. This compression and the resulting slight resilient deformation of the ribs helps to ensure a good seal for the e-liquid at the base of the cartomiser reservoir.

Also visible in Figure 5A, the atomising chamber 465 comprises four walls in a rectangular arrangement, a pair of opposing side walls 568, and a pair of opposing front and back walls 567. Each of the opposing side walls 568 includes a slot 569 which has an open end at the top (and in the centre) of the side wall, and a closed end 564 relatively near the bottom of the atomising chamber 465 - i.e. the two slots 569 extend more than halfway down their respective side walls 568.

Referring now to Figure 5B, this shows the wick/heater assembly 500 now fitted into the atomising chamber 465 of the cartomiser plug. In particular, the wick/heater assembly is positioned so that it extends between, and protrudes out of, the two opposing slots 569A, 569B. The wick is then lowered until it reaches the closed end 564 of each slot. Note that in this position, the coil 551 is located entirely in the atomizing chamber 465 - it is only the wick itself 440 that extends out of the slots into the reservoir area 270. It will be appreciated that this arrangement allows the wick to draw e-liquid from the reservoir 270 into the atomizing chamber 465 for vaporisation by the wire heater coil 551. Having the wick located near the bottom of the atomizing chamber, and more particularly also near the bottom of the reservoir 270, helps to ensure that the wick retains access to liquid in the reservoir even as the e-liquid is consumed, and hence the level of the e-liquid in the reservoir drops. Figure 5B also shows the heater contact wires 552A, 552B extending below the primary seal 460.

Figure 5C illustrates the underside of the base portion 462 of the primary seal 460. This view shows that the base portion includes two holes 582A, 582B, which are used for filing the reservoir 270 with e-liquid, as described in more detail below. The underside further includes a rectangular indentation 584 for receiving the PCB 470. A central hole 583 is provided in this indentation 584 to provide an air passage from underneath (and outside) the cartomiser into the atomisation (vaporisation) chamber 465. It will be appreciated that after assembly, this central hole 583 in the cartomiser plug is aligned with the corresponding central hole 471 in the PCB.

There are also two much smaller holes 587A, 587B formed in the rectangular indentation 584 of the lower portion of the cartomiser plug 460, one on either side of the central hole 583. The contact wires 552A and 552B extend downwards from the heater 450 and pass respectively through these two holes, 587A, 587B, in order to exit the vaporising chamber 465.

A slit 590A, 590B is formed in each of the front and back walls of the rectangular indentation 584. After extending through the two holes 587A, 587B, each contact wire from the heater is bent flat onto the underside of the cartomiser plug, and then leaves the rectangular indentation via the respective slits 590A, 590B. Thus contact wire 552A passes out of the atomising chamber 465 through hole 587A, and then exits the rectangular indentation 584 via slot 590A; likewise, contact wire 552B passes out of the atomising chamber 465 through hole 587B, and then exits the rectangular indentation 584 via slot 590B. The remaining portion of each wire 552A, 552B is then bent upwards towards the atomising chamber 465 in order to sit within a respective groove 597 in the cartomiser plug 460 (see Figure 5B). In some examples there may not be respective grooves 597 in the cartomiser plug 460 and the remaining portions of the each wire 552A, 552B may instead be simply bent to run alongside the side of cartomiser plug 460.

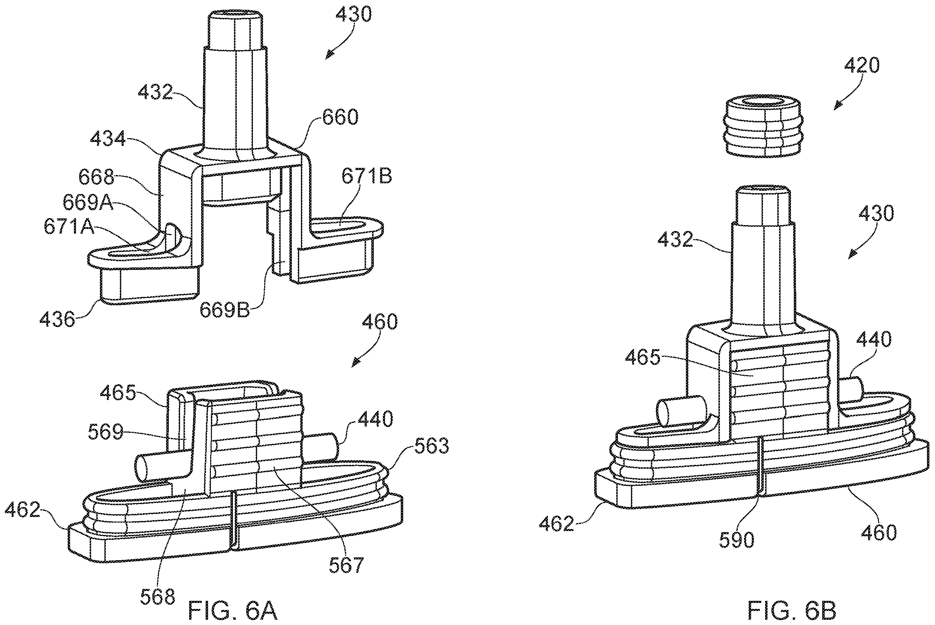

Figures 6A and 6B illustrate the inner frame and the vent seal being fitted into the cartomiser plug in accordance with some embodiments of the invention. Thus as previously described, the inner frame 430 comprises a base section 436, a middle section 434 and air tube 432 located at the top of the inner frame. The base section contains two slots 671A, 671B extending in a horizontal sideways direction (parallel to the x axis). As the base section 436 of the inner frame is lowered down past the atomizing chamber 465, the portions of the wick 440 that extend out from each side of the atomizing chamber 465 pass through these slots 671A, 671B, thereby allowing the base section of the inner frame to be lowered further until it is received in the lower portion 462 of the cartomiser plug.

As noted above, the middle section 434 of the inner frame complements and completes the atomizing chamber 465 of the cartomiser plug 460. In particular, the middle section provides two opposing side walls 668 and a top wall or roof 660. The latter closes the top of the atomizing chamber 465, except in respect of the air tube 432 which extends up from the atomizing chamber 465 to the exit hole 280 of the mouthpiece 250.

Each of the opposing side walls 668 includes a slot 669A, 669B which extends upwards (parallel to the y axis) from the bottom of the side wall to the closed end of the respective slot. Accordingly, as the base section 436 of the inner frame is lowered down past the atomizing chamber 465, the portions of the wick 440 that extend out from each side of the atomizing chamber 465 pass through these slots 669A, 669B (in addition to slots 671A, 671B). This therefore allows the side walls 668 of the inner frame 430 to overlap the side walls 568 of the cartomiser plug. Further downward movement of the inner frame 430 is prevented once the closed end of slots 669A, 669B contacts the wick 440, which coincides with the base section 436 of the inner frame being received into the lower portion 462 of the cartomiser plug. At this stage, the combination of cartomiser plug 460, heater/wick assembly 500, and inner frame 430, as shown in Figure 6B has been formed, and the vent seal 420 can now be fitted onto the air tube (pipe) 432 of the inner frame 430.

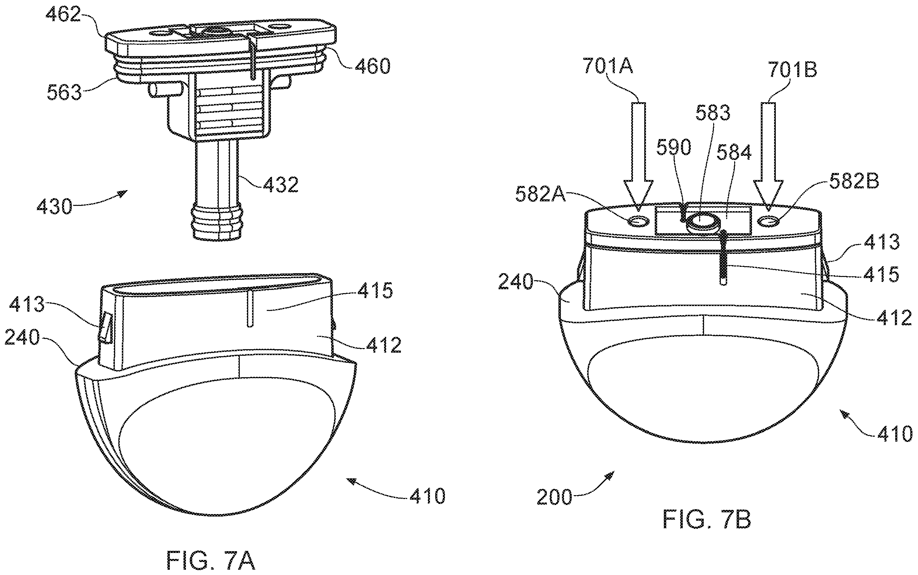

Figure 7A illustrates the combination of the inner frame 430, wick/heater assembly 500, and primary seal 460 being fitted into the shell 410. As this insertion occurs, the slot 415 in each of the front and back faces of the lower portion 412 of the shell 410 accommodates a portion of wire 552 that has passed through slot 590 and has been wrapped back up around the outside of the cartomiser plug 460 and into groove 597. Furthermore, the deformable ribs 563 around the lower portion 462 of the primary seal are slightly compressed by the inside wall of the lower portion 412 of the shell 410 during the insertion, and thereby form a seal to retain the e-liquid in the resulting reservoir 270. Accordingly, as illustrated in Figure 7B, the cartomiser 200 is now ready for filling with the e-liquid. This filling is performed, as indicated by arrows 701A, 701B, through holes 582A and 582B in the primary seal 460, and through slots 671A, 671B in the inner frame (not visible in Figure 7B).

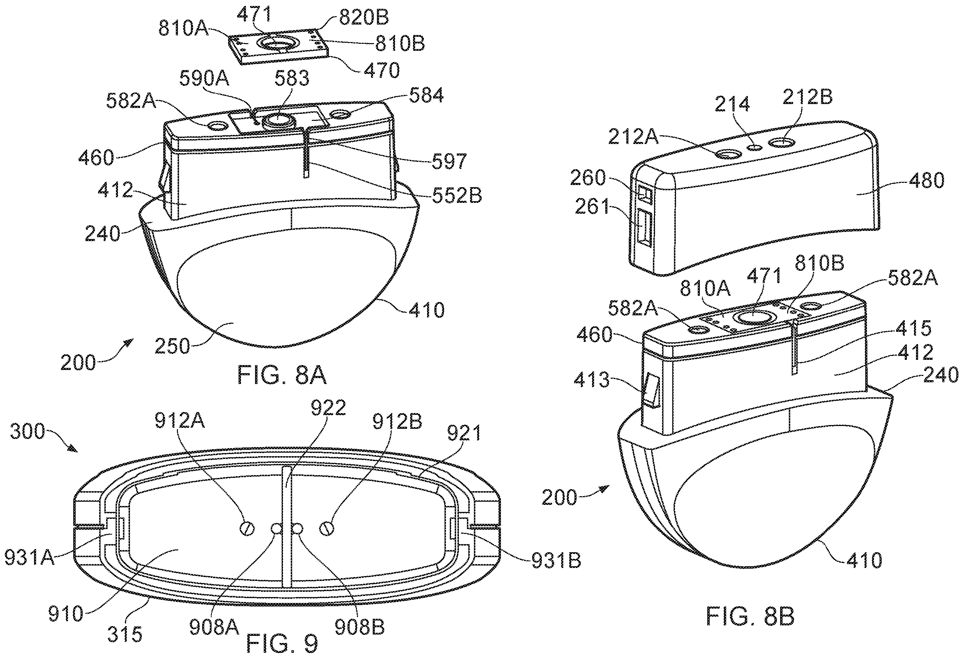

Figure 8A illustrates the PCB 470 being fitted into the rectangular indentation 584 in the underside of the primary seal 460. This fitting aligns the central hole 471 in the PCB with the central hole 583 in the primary seal 460 in order to provide the main airflow channel into the cartomiser 200.

As previously described, the rectangular indentation 584 is provided with a pair of holes 587, located on either side of the central hole 583. Each hole allows egress of a respective contact wire 552A, 552B from the vaporiser chamber 465. The contact wires 552A, 552B are bent flat against the floor of the rectangular indentation 584, and then exit the rectangular indentation 584 via respective slots 590A, 590B in the front and back walls of the rectangular indentation. The final portion of each heater contact wire 552A, 552B, is then bent upwards, back towards the top of the cartomiser and mouthpiece 250, and located in a corresponding groove or channel 597 formed in the cartomiser plug. In addition, the base portion of the shell also includes a slot 415 on each of the front and back faces to accommodate a respective heater contact wire 552A, 552B.

In accordance with some embodiments, the PCB 470 does not contain any active components, but rather provides two large contact pads 810A, 810B on either side of the central hole 471. These contact pads are visible in Figure 8A on the lower face of the PCB, i.e. the side facing the control unit 300 after assembly. The opposite face of the PCB, i.e. the upper side which is received into the rectangular indentation 584 and faces the heater 450, is provided with a similar, corresponding configuration of contact pads (not visible in Figure 8A). The heater contact wires 552A, 552B are in physical, and hence electrical, contact with a respective contact pad on the upper side of the PCB.

The opposing pairs of contact pads on either side of the PCB 470 are connected by respective sets of one or more vias 820A, 820B. In other words, vias 820A provide a conductive path between one contact pad on the lower face of the PCB and a corresponding contact pad on the upper face of the PCB, and vias 820B provide a conductive path between the other contact pad on the lower face of the PCB and its corresponding contact pad on the upper face of the PCB. Accordingly, when the control unit is connected to the cartomiser, pins from the control unit touch the contact pads on the lower side of the PCB 470, and electrical current flows to/from the heater 450 through the respective vias, contact pads on the upper side of the PCB 470, and respective heater contact wires 552A, 552B.

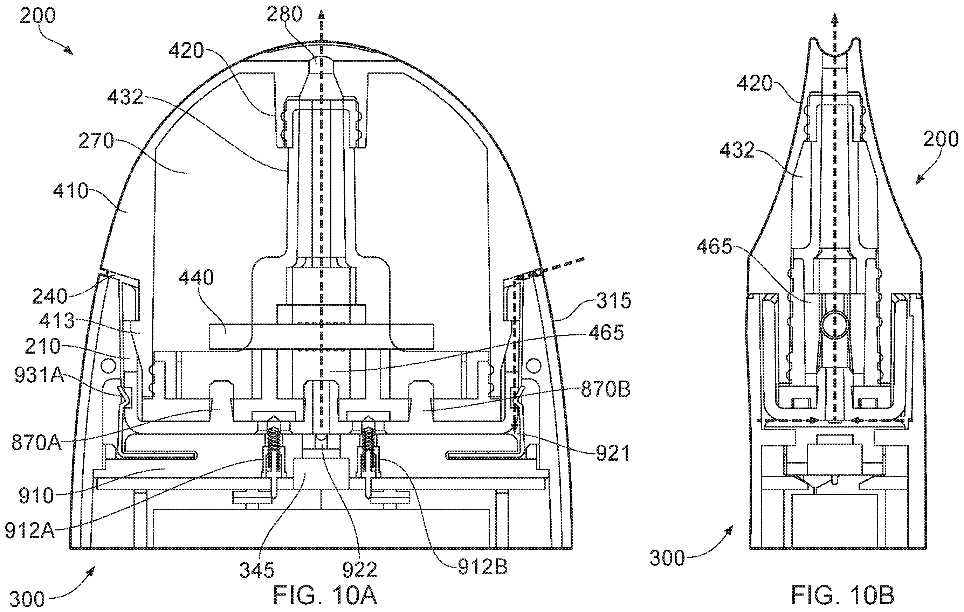

Figure 8B illustrates the end cap 480 being fitted to the cartomiser 200 in accordance with some embodiments of the invention. In particular, the end cap 480 is fitted over the end of the cartomiser plug 460 and the lower section 412 of the shell 410, and is retained in this position by the protruding member 413 provided on each side of the lower section 412 of the shell engaging into the corresponding hole or slot 261 on each side of the end cap. In this fully assembled state (see Figure 2), the end cap 480 covers and therefore closes the holes 582A, 582B in the cartomiser plug that were used for filling the liquid reservoir 270. Indeed, as can be seen in Figure 10A, the end cap 480 is provided with two upwardly directed plugs 870A and 870B that respectively penetrate and close the filling holes 582A, 582B. Accordingly, the reservoir 270 is now fully sealed, apart from the opening on each side of the atomising chamber 465 through which the wick 440 passes into the atomising chamber 465.

As previously discussed, the end cap includes three holes, a central hole 214 and two holes 212A, 212B located on either side of this central hole. The fitting of the end cap 480 aligns the central hole 214 of the end cap with the central hole 471 in the PCB and with the central hole 583 in the primary seal 460 in order to provide the main airflow channel into the cartomiser 200. The two side holes 212A, 212B allow pins from the control unit 300, acting as positive and negative terminals, to pass through the end cap 480 and make contact with respective contact pads 810A, 810B on the lower side of the PCB, thereby enabling the battery 350 in the control unit 300 to supply power to the heater 450.

In accordance with some embodiments, the primary seal 460, which as noted above is made of a resilient deformable material such as silicone, is held in a compressed state between the inner frame 430 and the end cap 480. In other words, the end cap is pushed onto the cartomiser 200 and compresses the primary seal 460 slightly before the latch components 413 and 261 engage with one another. Consequently, the primary seal remains in this slightly compressed state after the end cap 480 and shell 410 are latched together. One advantage of this compression is that the end cap acts to push the PCB 470 onto the heater contact wires 552A, 550B, thereby helping to ensure a good electrical connection without the use of solder.

Figure 9 is a top view looking down onto the control unit 300 of the e-cigarette of Figure 1 in accordance with some embodiments of the invention. The control unit includes external walls 315 that rise above the rest of the control unit (as best seen in Figure 1) to define a cavity for accommodating the lower portion 210 of the cartomiser. Each side of these walls 315 is provided with a spring clip 931A, 931B that engages with the hole or slot 260 on each side of the cartomiser 200 (see Figure 2), thereby retaining the cartomiser in engagement with the control unit 300 to form the assembled e-cigarette 100.

At the bottom of the cavity formed by the upper portion of control unit walls 315 (but otherwise at the top of the main body of the control unit 300) is a battery seal 910 (see also Figure 1). The battery seal 910 is formed from a resilient (and compressible) material such as silicone. The battery seal 910 helps to mitigate one potential risk with an e-cigarette 100, which is that e-liquid leaks from the reservoir 270 into the main air passage through the device (this risk is greater where there is free liquid in the reservoir, rather than the liquid being held by a foam or other such material). In particular, if e-liquid were able to leak into the portion of the control unit containing the battery 350 and control electronics, then this might short circuit or corrode such components. Furthermore, there is also a risk that the e-liquid itself would then become contaminated before returning into the cartomiser 200 and then exiting through the mouthpiece hole 280. Accordingly, if any e-liquid does leak into the central air passage of the cartomiser, the battery seal 910 helps to prevent such leakage progressing into the portion of the control unit that contains the battery 350 and control electronics. (The small holes 908 in the battery seal 910 do provide very limited fluid communication with the microphone 345 or other sensor device, but the microphone 345 itself can then act as a barrier against any such leakage progressing further into the control unit.

As shown in Figure 9, there is a small groove or spacing 921 around the perimeter between the top of the battery seal 910 and the inside of the walls 315 of the control unit; this is primarily formed by the rounded corner of the battery seal 910. The battery seal is further provided with a central groove 922 from front to back, which connects at both ends (front and back) with the perimeter groove 921 to support airflow into the cartomiser, as described in more detail below. Immediately adjacent to central groove 922 are two holes 908A, 908B, one on either side of the groove 922. These air holes extend down to the microphone 345. Thus when a user inhales, this causes a drop in pressure within the central air passage through the cartomiser 200, as defined by air tube 432, the central hole 583 in the primary seal 460, etc, and also within the central groove 922, which lies at the end of this central air passage. The drop in pressure further extends through holes 908A, 908B to the microphone 345, which detects the drop in pressure, and this detection is then used to trigger activation of the heater 450.

Also shown in Figure 9 are two contact pins, 912A, 912B, which are linked to the positive and negative terminals of the battery 350. These contact pins 912A, 912B pass through respective holes in the battery seal 910 and extend through holes 212A, 212B of the end cap to make contact with contact pads 810A, 810B respectively on the PCB. Accordingly, this then provides an electrical circuit for supplying electrical power to the heater 450. The contact pins may be resiliently mounted within the battery seal (sometimes referred to as "pogo pins"), such that the mounting is under compression when the cartomiser 200 is latched to the control unit 300. This compression causes the mounting to press the contact pins against the PCB contact pads 810A, 810B, thereby helping to ensure good electrical connectivity. It will be appreciated that approaches other than using pogo pins could be used. For example, in some cases the contact pins may not be spring mounted, but may instead accommodate a degree of resilient deflection when assembled to facilitate a biased contract with the PCB contact pads. In another cases, the contact pins may themselves be rigid and carried by a resiliently mounted support.

The battery seal 910, which as noted above is made of a resilient deformable material such as silicone, is held in a compressed state between the cartomiser 200 and the control unit 300. In other words, inserting the cartomiser into the cavity formed by walls 315 causes the end cap 480 of the cartomiser to compress the battery seal 910 slightly before the spring clips 931A, 931B of the control unit engage with the corresponding holes 260A, 260B in the lower portion 210 of the cartomiser. Consequently, the battery seal 910 remains in this slightly compressed state after the cartomiser 200 and the control unit 300 are latched together, which helps to provide protection against any leakage of e-liquid, as discussed above.

Figures 10A and 10B are cross-sections respectively (a) from side to side, and (b) from front to back, showing the airflow through the e-cigarette of Figure 1 in accordance with some embodiments of the invention. The airflow is denoted in Figures 10A and 10B by the heavy black, dashed arrows. (Note that Figure 10A only shows air flow on one side of the device, but there is an analogous air flow on the other side as well - having multiple such air inlets reduces the risk that a user will accidentally block the air inlets with their fingers while holding the device).

The airflow enters through a gap at the sides of the e-cigarette 100, in between the top of the walls 315 of the control unit, and the flange or rim 240 of the cartomiser shell 410. The airflow then passes down a slight spacing between the inside of the walls 315 and the outside of the lower portion 210 of the cartomiser 200, past the spring clips 931, and hence into perimeter groove 921 (as shown in Figure 9). The airflow is then drawn around the perimeter groove 921, and hence out of the plane of Figures 10A and 10B (so that this portion of the airflow path is therefore not visible in these two diagrams). Note that there is typically some space above the groove 921 between the inside of the control unit walls and the outside of the cartomiser end cap, so the airflow is not necessarily constrained to the groove 921 per se.

After travelling an angle of approximately 90 degrees around the perimeter groove 921, the airflow passes into the central groove 922, from where it travels to and through the central hole 583 of the end cap 480 and hence into the central air passage of the cartomiser. Note that Figure 10B shows this airflow along the central groove 922 into the central air passage, and then the flow of air up through the central air passage is shown in both Figures 10A and 10B. In contrast to groove 921, the space above groove 922 is not open, but rather the battery seal 910 is compressed against the end cap 480 of the cartomiser 200. This configuration results in the end cap covering the groove to form a closed channel having a confined space. This confined channel can be utilised to help control the draw resistance of the e-cigarette 100, as described in more detail below.

There are various benefits associated with the overall airflow path such as shown in Figures 10A and 10B. The airflow detector, such as microphone 345, is generally located in the control unit 300. This reduces cost because the microphone is therefore in the reusable portion of the device, and so there is no need to include a microphone in every cartomiser (the disposable component). In addition, having the microphone 345 in the control unit 300 allows the microphone to be readily connected to the battery 350 and to the control processor of the control unit (not shown in the Figures).

On the other hand, it is generally desirable to reduce or avoid an airflow past electronics components, for example, because such electronics components tend to become warm with use, and may potentially shed volatiles. It will be appreciated that the airflow path shown in Figures 10A and 10B largely bypasses the electronic components of the control unit, with only the small holes 908 branching off this main airflow to allow the microphone 345 to detect a change in pressure. This avoidance of airflow past the main electronic components of the control unit has been achieved despite the fact that the cartomizer sits quite deeply within the control unit (which helps to reduce the overall length of the device).

Furthermore, in many existing e-cigarettes, the overall air path is not tightly controlled. For example, air may leak into the air path at joins between various components (such as between the cartomiser and control unit), rather than just at the dedicated air inlet(s). This leakage (as well as various other manufacturing variations) may result in significant variation in the draw resistance of the device, where the draw resistance in effect represents the pressure difference needed to produce a given air flow through the device. This variation in draw resistance can prevent a consistent user experience and can also effect the operation of the device. For example, if the draw resistance is high, it is likely that the flow of air through the device may be reduced, which in turn reduces the amount of air cooling experienced by the heater.

Accordingly, the approach described herein provides an e-cigarette device including: an atomiser for vaporising a liquid; an air passage through the atomiser, the air passage exiting the e-cigarette at a mouthpiece; at least one air inlet joined by a channel to the air passage through the vaporiser; and at least one resilient seal which acts to prevent air from the air inlet travelling to the air passage except through the channel.

For example, in the implementation described above, the air flow entering the central air passage through the vaporiser must first travel along groove 922. This groove, in conjunction with the bottom of the end cap 480 that in effect provides a top surface or closure for the groove, defines the airflow channel through the control unit into the cartomiser.

In such a device, air from the air inlet must necessarily travel through the channel to reach the air passage (because the seal prevents other routes). Accordingly, the channel provides a point of control for the draw resistance - especially if the channel provides the majority of the draw resistance for the air path through the whole device. In particular, as long as the draw resistance for the channel (which is determined largely by the size of the channel) is reasonably constant between devices (and between different usages of the same device), then the draw resistance for the device as a whole will likewise be reasonably constant.

In some implementations, the e-cigarette may further comprise a facility to alter the predetermined draw resistance for the e-cigarette. This facility may allow a user to set the predetermined draw resistance for the e-cigarette to one of a limited number of discrete values according to individual preference, etc. For example, for the e-cigarette described herein, there may be two successive latch positions between the cartomiser 200 and the control unit 300, which result in a lower or greater compression of the battery seal 910. The lower compression will generally allow groove 922 to expand slightly, and hence provide a lower draw resistance than the latch position which produces the higher compression of the battery seal. Another way of implementing this facility would be to provide some baffle that can be moved into the channel or groove 922 to partly obstruct the airflow by a desired amount.

The seal may be formed of a resilient material, such as silicone, and the channel is formed at least in part by the seal material itself. For example, in some embodiments, the channel is defined by a resilient material compressed against a surface of a rigid material, such as the battery seal 910 pressing against the end cap 480, and the surface of the rigid material may include a hole, such as hole 583 in end cap 480, that connects from the channel 922 into the air passage through the atomiser. Note that the channel may in fact comprises a network of multiple (sub)channels as appropriate, according to the particular implementation.

As described above, the device may include a cartomiser 200 and a control unit 300, and the resilient seal is provided as part of the control unit that contacts the exterior of the cartomiser when the cartomiser is joined to the control unit. The resilient material may be held under compression between the cartomiser and the control unit when the cartomiser is joined to the control unit, such as by a latch mechanism. This compression of the resilient material helps to provide an air-tight seal around the edges of the seal.

A further consideration is that for some e-cigarettes, there is a risk that the e-liquid may leak 270 into main air passage. In such a situation, the seal helps to ensure that the e-liquid is only able to travel from the air passage into the air channel, thereby helping to prevent the e-liquid coming into contact with the battery and other electrical components. Furthermore, the air channel may be sufficiently narrow to prevent significant flow of e-liquid through the channel, which further helps to constrain any leaked e-liquid.

Although various embodiments have been described in detail herein, this is by way of example only, it will be appreciated that a channel to constrain airflow into a device may be utilised in many different configurations. For example, this approach might be used for a one-piece or three-piece device (rather than a two-piece device, i.e. cartomiser and control unit, as described here). Similarly, this approach could be utilised with electronic vapour provision systems that includes material derived from tobacco plants which is provided in any suitable form (powder, paste, shredded leaf material, etc, i.e. not liquid), and then heated to produce volatiles for inhalation by a user. This approach could also be used with various types of heater for the e-cigarette, various types of airflow configuration, various types of connection between the cartomiser and the control unit (such as screw or bayonet) etc. The skilled person will be aware of various other forms of electronic vapour provision system which might utilise a channel for restricting the airflow as described herein.

Furthermore, it will be appreciated the manner of cartomiser assembly set out above is merely one example, and an assembly process comprising different steps, or a similar steps performed in a different order may also be adopted. For example, with reference to the steps set out in relation to Figures 6B, 7A and 7B, in another example instead of fitting the vent seal 420 to the air tube (pipe) 432 of the inner frame (Figure 6B) before placing the combined assembly in the shell 410 (Figures 7A and 7B), the vent seal 420 might first be mounted in position in the shell 410 so that it mounts to the air tube (pipe) 432 of the inner frame when the inner frame 430, wick/heater assembly 500, and primary seal 460 are together fitted into the shell 410. Similarly, with reference to the steps set out in relation to Figures 8A and 8B, in another example instead of placing the PCB 470 in its indentation 584 in the cartomiser plug 460 before attaching the cap 480 to complete the cartomiser assembly, the PCB 470 might first be mounted in position in the cap 480, and then the cap 480, with PCB 470 attached, connected to the shell 410. The PCB 470 may mount to the cap 480 by a friction / press fit, for example. The cap may include locating pegs, or other guide mechanism, to help position the PCB in the cap so it is aligned with the indentation 584 in the cartomiser plug when the cap is attached to the shell.

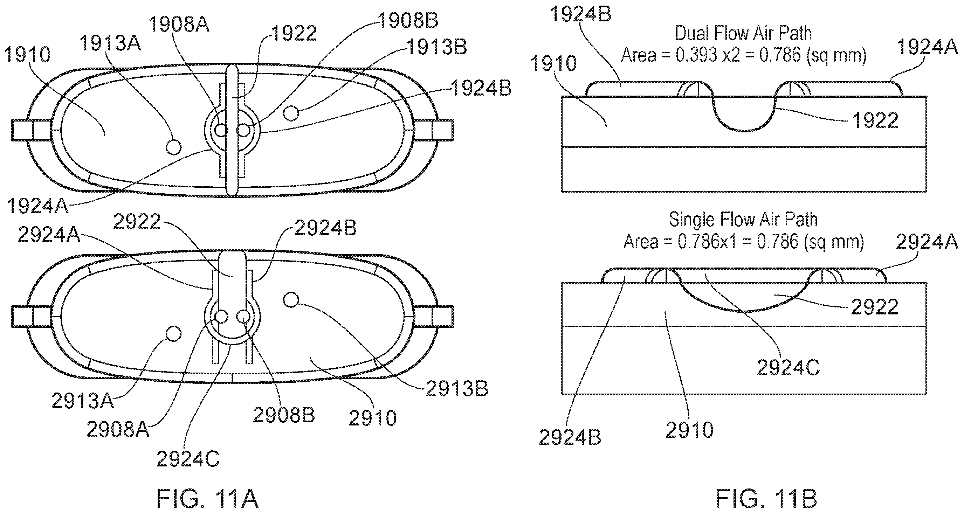

Figure 11A shows two alternative implementations (upper and lower) of the battery seal in accordance with some embodiments of the invention. In particular, Figure 11A shows a top view of the battery seal, analogous to that of Figure 9, except in Figure 11A the two battery seals are shown in isolation, i.e. not fitted into a control unit 300. The upper battery seal of Figure 11A is denoted by reference number 1910, the lower battery seal of Figure 11A is denoted by reference number 2910. Battery seals 1910, 2910 generally may be made of the same material, and perform the same function, as battery seal 910 described above. These shared aspects will not be described again here (or will be described here only briefly) to reduce or avoid repetition.

As shown in Figure 11A, battery seal 1910 includes a pair of holes 1908A, 1908B, corresponding to holes 908A, 908B for battery seal 910, to allow a microphone 345 (see Figure 10A) to detect an inhalation. Battery seal 2910 includes a similar pair of holes 2908A, 2908B for performing the same function. Battery seal 1910 also includes a pair of holes 1913A, 1913B, likewise battery seal 2910 includes a pair of holes 2913A, 2913B. These holes are used to receive contact pins, such as pins 912A and 912B as shown in Figure 9, which in turn make contact with pads 810A, 810B on the PCB 470 (see Figures 8A and 8B) to provide power from the battery in the control unit 300 to the atomiser. For design reasons, the holes 1913A, 1913B and 2913A, 2913B are slightly offset from the positions of pins 912A, 912B, but remain rotationally symmetrical, as for the implementation of Figure 9; the cartomiser 200 can therefore still be joined to the control unit 300 in two different orientations, separated from one another by a rotation of 180° about the Y-axis of Figure 2.

The channel 1922 of battery seal 1910, as shown in Figure 11A top, is similar to the channel 922 of battery seal 910, as shown in Figure 9. In particular, the channels 922, 1922 stretch across the short dimension of the planar surface, joining the perimeter of the battery seal to the central air passage (corresponding to through hole 471 in PCB 470). One way to view channels 922, 1922 is that each is formed of two sub-channels, a first subchannel from the perimeter to the central air passage, and a second subchannel, also from the perimeter to the central air passage, but in an opposite location to the first subchannel. The volume of air reaching the central air passage into the cartomiser 200 is therefore the combination of the air travelling along these first and second channels.

The channel 2922 of battery seal 2910, as shown in Figure 11A bottom, is different from the channel 922 of battery seal 910 (and from channel 1922 of battery seal 1910), in that channel 2922 does not comprise two separate subchannels - rather channel 2922 provides just a single path from the perimeter to the central air passage.

Figure 11B shows a cross-section through each of the channels shown in Figure 11A. More particularly Figure 11B top is a cross-section through the battery seal 1910 shown in Figure 11A top, and Figure 11B bottom is a cross-section through the battery seal 2910 shown in Figure 11A bottom. It can be seen that the cross-sectional area through channel 2922 in battery seal 2910 is significantly greater than the cross-sectional area through channel 1922 in battery seal 1910 (which is also approximately the same size as the channel 922 in battery seal 910).

In the particular implementations shown, the cross-sectional area through channel 2922 in battery seal 2910 is approximately twice the cross-sectional area through channel 1922 in battery seal 1910. Since channel 1922 in battery seal 1910 comprises two separate paths (subchannels) from the perimeter to the central air passage, whereas channel 2922 in battery seal 2910 comprises only a single path (subchannel) from the perimeter to the central air passage, the overall cross-sectional area for airflow from the perimeter to the central air passage is therefore approximately the same for both channel 2922 in battery seal 2910 and channel 1922 in battery seal 1910. This is confirmed by the specific measurements of cross-sectional area given in Figure 11B (by way of example), whereby the single flow air path of Figure 11B bottom has a cross-sectional area of 0.786 mm², the same as the composite value for the dual flow air path of Figure 11B top.

The increase in size of the single flow air path of Figure 11B bottom compared with the size of an individual subchannel of the dual flow air path of Figure 11A top is primarily due to an increase in width of the former compared to the latter (the channel depth is approximately the same for both cases). Since the total cross-sectional area for air-flow is the same (approximately) for both the single and dual air flow paths of Figure 11B bottom and top respectively, the overall resistance to draw (RTD) is similar for both implementations. However, it has been found that the use of two smaller subchannels for the dual air flow path of Figure 11B top is more susceptible to producing unwanted noise during an inhalation, as the air is drawn through the smaller subchannels. In contrast, the single air flow path of Figure 11B bottom has been found to be less susceptible to producing such unwanted noise during an inhalation. One possible reason for this is that the smaller subchannels of Figure 11B top may be more prone to edge effects causing turbulence, etc.

Overall, this leads to various considerations for the sizing of the (sub)channels - in particular, to provide the desired RTD, to avoid or reduce the risk of any liquid which has not been vaporised exiting along the air channel 922, and to avoid or reduce noise during inhalation. The exact sizing to meet these differing objectives is dependent on the precise configuration of any given implementation, but typically the cross-sectional area of each subchannel is likely to be in range 0.3-1.5 mm², more typically in the range 0.5-1.0 mm², and the cross-sectional area of the overall channel (which may be formed of multiple subchannels) is typically in the range in range 0.5-1.5 mm², more typically in the range 0.6-1.0 mm².

A further difference between the battery seal 910 of Figure 9 and the battery seals 1910 and 2910 shown in Figure 11A is that the latter are provided with a ridge to improve the air seal formed by the channel. Thus with reference to Figure 11A top, the battery seal 1910 comprises two ridges 1924A, 1924B, one on each side of the channel 1922. As can be seen in Figure 11B top, these ridges 1924A and 1924B protrude above the planar surface of the battery seal 1910. The ridges are formed integrally with the battery seal (and of the same resilient material). The height of the ridge is less than the depth of the channel.

When the control unit 300 is assembled to the cartomiser 200, the battery seal is pressed against the underside of the cartomiser 200. In particular, the battery seal slightly deforms in the region of the ridge, since the ridge 1924 is the portion of the battery seal that extends furthest towards the cartomiser, and hence the base of the cartomiser 200, in effect, pushes the ridge down into the battery seal. This resilient deformation of the battery seal therefore results in an opposing force that causes the ridge 1924 to be pushed or held tightly against the underside of the cartomiser, thereby providing a more secure air seal along the channel.

In battery seal 1910 (Figure 11A, top), the ridge is formed of two components 1924A, 1924B, which are generally adjacent to opposing sides of the channel to help provide the air seal for the channel. Note that the ridge components 1924A, 1924B curve around air holes 1908A, 1908B. During an inhalation which draws in air along channel 1922, this shaping of the ridges 1924A, 1924B allows the air holes 1908A, 1908B to experience the pressure reduction caused by the inhalation, and hence the microphone can detect the inhalation.

The battery sea 2910 (Figure 11A, bottom) includes a similar ridge to battery seal 1910. In particular, battery seal 2910 contains ridge components 2924A, 2924B that are aligned with and adjacent to the channel 2922 on opposing sides of the channel. As for battery seal 1910, the ridge extends around the outside of air holes 2908A, 2908B, to enable an inhalation to be detected through these holes. The ridge of battery seal 2910 further includes an additional component 2924C, which blocks off the end of the channel, i.e. on opposite side of the central air passage to the side on which channel 2922 is located. It will be appreciated that this configuration, with the addition of ridge component 2924C, completes the air seal around the channel 2922.

In conclusion, in order to address various issues and advance the art, this disclosure shows by way of illustration various embodiments in which the claimed invention(s) may be practiced. The advantages and features of the disclosure are of a representative sample of embodiments only, and are not exhaustive and/or exclusive. They are presented only to assist in understanding and to teach the claimed invention(s). It is to be understood that advantages, embodiments, examples, functions, features, structures, and/or other aspects of the disclosure are not to be considered limitations on the disclosure as defined by the claims or limitations on equivalents to the claims, and that other embodiments may be utilised and modifications may be made without departing from the scope of the claims. Various embodiments may suitably comprise, consist of, or consist essentially of, various combinations of the disclosed elements, components, features, parts, steps, means, etc other than those specifically described herein. The disclosure may include other inventions not presently claimed, but which may be claimed in future.

## Claims

1. A vapour provision device (100) including:
an atomiser for vaporising a liquid;
an air passage through the atomiser, the air passage exiting the vapour provision device at a mouthpiece (250);
at least one air inlet, joined by a channel (921, 922) to the air passage through the atomiser; and
at least one resilient seal (910) which acts to restrict air from the air inlet travelling to the air passage except through the channel;
wherein the seal comprises a resilient material;
and **characterised in that** the size of the channel is defined at least in part by the resilient material to control resistance to draw for the vapour provision device.

2. The device of claim 1 wherein the seal is provided at least in part where the channel joins the air passage through the atomiser.

3. The device of claim 1 or 2, wherein the channel is defined by a resilient material compressed against a surface (480) of a rigid material, and optionally wherein the surface of the rigid material includes a hole (214) that connects from the channel into the air passage through the atomiser

4. The device of claim 3, wherein the device includes a cartomiser (200) that contains the atomiser, and the surface of the rigid material is part of the exterior of the cartomiser.

5. The device of claim 4, wherein the device further includes a control unit (300) that contains a battery (350) for powering the e-cigarette, and the resilient seal is provided as part of the control unit that contacts the exterior of the cartomiser when the cartomiser is joined to the control unit, and optionally wherein the resilient material is held under compression when the cartomiser is joined to the control unit, and optionally further comprising a latch mechanism (260, 931A, 931B) for joining the cartomiser to the control unit to hold the resilient material under compression.

6. The device of claim 5, wherein the resilient seal further acts to restrict leakage of the liquid from travelling through the air passage into contact with the battery and/or wherein the at least one air inlet is located at the junction between the cartomiser and the control unit.

7. The device of any of claims 3 to 6, wherein the surface of the rigid material is substantially planar, and the resilient material comprises a planar surface which is compressed against the planar surface of the rigid material, and optionally wherein the channel is defined within the planar surface of the rigid material or of the resilient material.

8. The device of claim 7, wherein the device comprises a central longitudinal axis which is perpendicular to the planar surface of the resilient material and the planar surface of the rigid material, and wherein the air passage is substantially aligned with said central longitudinal axis, and optionally wherein the channel extends from a perimeter of the planar surface of the resilient material to the central longitudinal axis for joining to the air passage.

9. The device of claim 7 or 8, wherein the resilient seal further comprises at least one ridge (1924A, 1924B) that protrudes from planar surface of one of the resilient or rigid material to contact the planar surface of the other of the resilient or rigid material, and optionally wherein the at least one ridge is aligned with and adjacent to the channel.

10. The device of any claims 1 to 9, wherein the channel is formed of a plurality of subchannels, and/or wherein the channel comprises a groove formed in a surface of the resilient material, and/or wherein the resilient seal is made from silicone.

11. The device of any of claims 1 to 10, wherein the channel is configured to provide a predetermined draw resistance, and optionally comprising a mechanism which can be set by a user to alter the predetermined draw resistance, and optionally wherein the mechanism which can be set by a user to alter the predetermined draw resistance to one of a limited number of discrete values.

12. The device of any of claims 1 to 11, wherein multiple air inlets are provided on the surface of the device, and wherein said multiple air inlets are all joined by the channel to the air passage through the atomiser, and/or wherein the draw resistance through the channel is greater than the draw resistance through the air passage.

13. The device of any of claims 1 to 12, wherein the channel has a cross-sectional area which is sufficiently small as to restrict any liquid which has leaked into the air passage from flowing down the channel, and/or wherein the channel has a sufficiently large cross-sectional area to allow air to travel through the channel during an inhalation by a user without creating audible noise for the user, and/or wherein the channel has a cross-sectional area in the range 0.5 mm² to 1.5 mm², preferably in the range 0.6 mm² to 1.0 mm²

14. The device of any of claims 1 to 13, wherein the air passage through the atomiser and to the mouthpiece is located along a central longitudinal axis of the device, and/or wherein the channel joins the air passage through the atomiser upstream of the atomiser and/or wherein the channel is joined to the air inlet which is located on an external surface of the device.

15. The device of any of claims 1 to 14, wherein the resilient seal is spaced from the liquid for vaporisation, such that the liquid does not contact the resilient seal, and/or further comprising a reservoir (270) of the liquid for vaporisation by the atomiser, said reservoir being located around the air passage.

## Patentansprüche

1. Dampfbereitstellungsvorrichtung (100), umfassend:
einen Atomizer zum Verdampfen einer Flüssigkeit;
einen Luftdurchlass durch den Atomizer, wobei der Luftdurchlass die Dampfbereitstellungsvorrichtung an einem Mundstück (250) verlässt;
mindestens einen Lufteinlass, der durch einen Kanal (921, 922) mit dem Luftdurchlass über den Atomizer verbunden ist; und
mindestens eine elastische Dichtung (910), die dazu dient, die Luft aus dem Lufteinlass, die in den Luftdurchlass wandert, mit Ausnahme des Kanals, zu begrenzen;
wobei die Dichtung ein elastisches Material umfasst;
und **dadurch gekennzeichnet, dass** die Größe des Kanals mindestens teilweise durch das elastische Material definiert ist, um den Zugwiderstand der Dampfbereitstellungsvorrichtung zu steuern.

2. Vorrichtung nach Anspruch 1, wobei die Dichtung mindestens teilweise dort bereitgestellt ist, wo sich der Kanal über den Atomizer mit dem Luftdurchlass verbindet.

3. Vorrichtung nach Anspruch 1 oder 2, wobei der Kanal durch ein elastisches Material definiert wird, das gegen eine Oberfläche (480) eines starren Materials gedrückt wird, und wobei optional die Oberfläche des starren Materials ein Loch (214) umfasst, das von dem Kanal durch den Atomizer in den ersten Luftdurchlass führt.

4. Vorrichtung nach Anspruch 3, wobei die Vorrichtung einen Cartomizer (200) umfasst, der den Atomizer enthält, und die Oberfläche des starren Materials Teil der Außenseite des Cartomizers ist.

5. Vorrichtung nach Anspruch 4, wobei die Vorrichtung ferner eine Steuereinheit (300) umfasst, die eine Batterie (350) zum Betreiben der E-Zigarette enthält, und die elastische Dichtung als Teil der Steuereinheit bereitgestellt ist, die mit der Außenseite des Cartomizers in Berührung kommt, wenn der Cartomizer mit der Steuereinheit verbunden ist, und wobei optional das elastische Material unter Druck gehalten wird, wenn der Cartomizer mit der Steuereinheit verbunden wird, und optional ferner umfassend einen Verriegelungsmechanismus (260, 931A, 931B) zum Verbinden des Cartomizers mit der Steuereinheit, um das elastische Material unter Druck zu halten.

6. Vorrichtung nach Anspruch 5, wobei die elastische Dichtung ferner dazu dient, ein Auslaufen der Flüssigkeit beim Wandern durch den Luftdurchlass in Kontakt mit der Batterie zu begrenzen und/oder wobei sich der mindestens eine Lufteinlass an der Verbindung zwischen dem Cartomizer und der Steuereinheit befindet.

7. Vorrichtung nach einem der Ansprüche 3 bis 6, wobei die Oberfläche des starren Materials im Wesentlichen eben ist und das elastische Material eine ebene Oberfläche umfasst, die gegen die ebene Oberfläche des starren Materials gedrückt wird, und wobei optional der Kanal in der ebenen Oberfläche des starren Materials oder des elastischen Materials definiert wird.

8. Vorrichtung nach Anspruch 7, wobei die Vorrichtung eine zentrale Längsachse umfasst, die senkrecht zu der ebenen Oberfläche des elastischen Materials und zu der ebenen Oberfläche des starren Materials ist, und wobei der Luftdurchlass im Wesentlichen auf die zentrale Längsachse ausgerichtet ist, und wobei sich optional der Kanal von einem Umfang der ebenen Oberfläche des elastischen Materials zur zentralen Längsachse zum Verbinden des Luftdurchlasses erstreckt.

9. Vorrichtung nach Anspruch 7 oder 8, wobei die elastische Dichtung ferner mindestens eine Wulst (1942A, 1942B) umfasst, die von der ebenen Oberfläche von einem des elastischen oder starren Materials vorsteht, um die ebene Oberfläche des anderen des elastischen oder starren Materials zu berühren, und wobei optional die mindestens eine Wulst auf den Kanal ausgerichtet ist und daran angrenzt.

10. Vorrichtung nach einem der Ansprüche 1 bis 9, wobei der Kanal aus einer Vielzahl von Unterkanälen gebildet ist, und/oder wobei der Kanal eine in einer Oberfläche des elastischen Materials gebildete Nut umfasst, und/oder wobei die elastische Dichtung aus Silikon besteht.

11. Vorrichtung nach einem der Ansprüche 1 bis 10, wobei der Kanal dazu ausgelegt ist, einen vorbestimmten Zugwiderstand bereitzustellen, und optional einen Mechanismus umfasst, der von einem Nutzer eingestellt werden kann, um den vorbestimmten Zugwiderstand zu verändern, und wobei optional der Mechanismus, der von einem Nutzer eingestellt werden kann, um den vorbestimmten Zugwiderstand auf einen von einer begrenzten Anzahl an bestimmten Werten zu verändern.

12. Vorrichtung nach einem der Ansprüche 1 bis 11, wobei mehrere Lufteinlässe an der Oberfläche der Vorrichtung bereitgestellt sind, und wobei die mehreren Lufteinlässe alle von dem Kanal bis zu dem Luftdurchlass über den Atomizer verbunden sind, und/oder wobei der Zugwiderstand durch den Kanal größer ist als der Zugwiderstand durch den Luftdurchlass.

13. Vorrichtung nach einem der Ansprüche 1 bis 12, wobei der Kanal eine Querschnittsfläche aufweist, die klein genug ist, um eine Flüssigkeit, die in den Luftdurchlass ausgetreten ist, davon abzuhalten,
den Kanal hinabzufließen, und/oder wobei der Kanal eine ausreichend große Querschnittsfläche aufweist, die es ermöglicht, dass bei einer Inhalation durch einen Nutzer Luft durch den Kanal strömt, ohne für den Nutzer hörbare Geräusche zu erzeugen, und/oder wobei der Kanal eine Querschnittsfläche im Bereich von 0,5 mm² bis 1,5 mm², vorzugsweise im Bereich von 0,6 mm² bis 1,0 mm² aufweist.

14. Vorrichtung nach einem der Ansprüche 1 bis 13, wobei sich der Luftdurchlass durch den Atomizer und zum Mundstück entlang einer zentralen Längsachse der Vorrichtung befindet, und/oder wobei der Kanal den Luftdurchlass über den Atomizer dem Atomizer vorgelagert verbindet und/oder wobei der Kanal mit dem Lufteinlass verbunden ist, der sich an einer Außenseite der Vorrichtung befindet.

15. Vorrichtung nach einem der Ansprüche 1 bis 14, wobei die elastische Dichtung von der Flüssigkeit zur Verdampfung beabstandet ist, sodass die Flüssigkeit nicht mit der elastischen Dichtung in Berührung kommt, und/oder ferner umfassend einen Behälter (270) mit der Flüssigkeit zur Verdampfung durch den Atomizer, wobei sich der Behälter um den Luftdurchlass herum befindet.

## Revendications

1. Dispositif de fourniture de vapeur (100) comprenant :
un atomiseur pour vaporiser un liquide ;
un passage d'air à travers l'atomiseur, le passage d'air sortant du dispositif de fourniture de vapeur au niveau d'un embout buccal (250) ;
au moins une entrée d'air, reliée par un canal (921, 922) au passage d'air à travers l'atomiseur ; et
au moins un joint d'étanchéité élastique (910) qui agit pour limiter le déplacement de l'air à partir de l'entrée d'air vers le passage d'air sauf à travers le canal ;
dans lequel le joint d'étanchéité comprend un matériau élastique ;
et **caractérisé en ce que** la taille du canal est définie au moins en partie par le matériau élastique pour commander la résistance au tirage pour le dispositif de fourniture de vapeur.

2. Dispositif selon la revendication 1, dans lequel le joint d'étanchéité est disposé au moins en partie là où le canal rejoint le passage d'air à travers l'atomiseur.

3. Dispositif selon la revendication 1 ou 2, dans lequel le canal est défini par un matériau élastique comprimé contre une surface (480) d'un matériau rigide et, facultativement, dans lequel la surface du matériau rigide comprend un trou (214) qui va depuis le canal dans le passage d'air à travers l'atomiseur.

4. Dispositif selon la revendication 3, dans lequel le dispositif comprend un cartomiseur (200) qui contient l'atomiseur et la surface du matériau rigide fait partie de l'extérieur du cartomiseur.

5. Dispositif selon la revendication 4, dans lequel le dispositif comprend en outre une unité de commande (300) qui contient une batterie (350) pour alimenter la cigarette électronique et le joint d'étanchéité élastique est fourni en tant que partie de l'unité de commande qui vient en contact avec l'extérieur du cartomiseur lorsque le cartomiseur est relié à l'unité de commande et, facultativement, dans lequel le matériau élastique est maintenu sous compression lorsque le cartomiseur est relié à l'unité de commande et, facultativement, comprenant en outre un mécanisme de verrouillage (260, 931A, 931B) pour relier le cartomiseur à l'unité de commande pour maintenir le matériau élastique sous compression.

6. Dispositif selon la revendication 5, dans lequel le joint d'étanchéité élastique agit en outre pour limiter le déplacement d'une fuite du liquide à travers le passage d'air en contact avec la batterie et/ou dans lequel la ou les entrées d'air sont situées au niveau de la jonction entre le cartomiseur et l'unité de commande.

7. Dispositif selon l'une quelconque des revendications 3 à 6, dans lequel la surface du matériau rigide est sensiblement plane et le matériau élastique comprend une surface plane qui est comprimée contre la surface plane du matériau rigide et, facultativement, dans lequel le canal est défini à l'intérieur de la surface plane du matériau rigide ou du matériau élastique.

8. Dispositif selon la revendication 7, le dispositif comprenant un axe longitudinal central qui est perpendiculaire à la surface plane du matériau élastique et à la surface plane du matériau rigide et dans lequel le passage d'air est sensiblement aligné sur ledit axe longitudinal central et, facultativement, dans lequel le canal s'étend depuis un périmètre de la surface plane du matériau élastique jusqu'à l'axe longitudinal central pour être relié au passage d'air.

9. Dispositif selon la revendication 7 ou 8, dans lequel le joint d'étanchéité élastique comprend en outre au moins une arête (1924A, 1924B) qui fait saillie depuis la surface plane de l'un parmi le matériau élastique ou le matériau rigide pour venir en contact avec la surface plane de l'autre parmi le matériau élastique ou le matériau rigide et, facultativement, dans lequel la ou les arêtes sont alignées sur le canal et adjacentes à celui-ci.

10. Dispositif selon l'une quelconque des revendications 1 à 9, dans lequel le canal est formé d'une pluralité de sous-canaux et/ou dans lequel le canal comprend une rainure formée dans une surface du matériau élastique et/ou dans lequel le joint d'étanchéité élastique est réalisé en silicone.

11. Dispositif selon l'une quelconque des revendications 1 à 10, dans lequel le canal est configuré pour fournir une résistance au tirage prédéterminée et, facultativement, comprenant un mécanisme qui peut être réglé par un utilisateur pour modifier la résistance au tirage prédéterminée et, facultativement, dans lequel le mécanisme qui peut être réglé par un utilisateur pour modifier la résistance au tirage prédéterminée à l'une d'un nombre limité de valeurs discrètes.

12. Dispositif selon l'une quelconque des revendications 1 à 11, dans lequel de multiples entrées d'air sont ménagées sur la surface du dispositif et dans lequel lesdites multiples entrées d'air sont toutes reliées par le canal au passage d'air à travers l'atomiseur et/ou dans lequel la résistance au tirage à travers le canal est plus importante que la résistance au tirage à travers le passage d'air.

13. Dispositif selon l'une quelconque des revendications 1 à 12, dans lequel le canal présente une surface de section transversale qui est suffisamment petite de sorte à limiter l'écoulement jusqu'au canal de n'importe quel liquide qui a fui dans le passage d'air et/ou dans lequel le canal présente une surface de section transversale suffisamment importante pour permettre à l'air de se déplacer à travers le canal pendant une inhalation par un utilisateur sans créer un bruit audible pour l'utilisateur et/ou dans lequel le canal présente une surface de section transversale dans la plage allant de 0,5 mm² à 1,5 mm², de préférence dans la plage allant de 0,6 mm² à 1,0 mm².

14. Dispositif selon l'une quelconque des revendications 1 à 13, dans lequel le passage d'air à travers l'atomiseur et jusqu'à l'embout buccal est situé le long d'un axe longitudinal central du dispositif et/ou dans lequel le canal rejoint le passage d'air à travers l'atomiseur en amont de l'atomiseur et/ou dans lequel le canal est relié à l'entrée d'air qui est située sur une surface externe du dispositif.

15. Dispositif selon l'une quelconque des revendications 1 à 14, dans lequel le joint d'étanchéité élastique est espacé du liquide pour une vaporisation de telle sorte que le liquide ne vienne pas en contact avec le joint d'étanchéité élastique et/ou comprenant en outre un réservoir (270) du liquide pour une vaporisation au moyen de l'atomiseur, ledit réservoir étant situé autour du passage d'air.
